Europäisches Patentamt

European Patent Office

Office européen des brevets

(11) Veröffentlichungsnummer: **0 272 414 B1**

# EUROPÄISCHE PATENTSCHRIFT

(45) Veröffentlichungstag der Patentschrift: **23.10.91**

(51) Int. Cl.5: **A61M 1/16**

(21) Anmeldenummer: **87116070.1**

(22) Anmeldetag: **31.10.87**

(54) **Vorrichtung zur Bestimmung der Veränderung des intravasalen Blutvolumens während der Hämodialyse.**

(30) Priorität: **24.11.86 DE 3640089**

(43) Veröffentlichungstag der Anmeldung:
**29.06.88 Patentblatt 88/26**

(45) Bekanntmachung des Hinweises auf die
Patenterteilung:
**23.10.91 Patentblatt 91/43**

(84) Benannte Vertragsstaaten:
**DE ES FR GB IT SE**

(56) Entgegenhaltungen:
**EP-A- 0 089 003**
**EP-A- 0 122 604**
**US-A- 4 197 196**

**WIENER KLINISCHE WOCHENSCHRIFT, 91.
Jg., Heft 22, 1979, Seiten 762-765; H. HOLZER
et al.: "Die kontinuierliche Messung der Blutdichte während der Hämodialyse"**

(73) Patentinhaber: **Fresenius AG**
**Gluckensteinweg 5**
**W-6380 Bad Homburg v.d.H.(DE)**

(72) Erfinder: **Polaschegg, Hans-Dietrich, Dr.**
**Grünwiesenweg 9**
**W-6370 Oberursel 4(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

EP 0 272 414 B1

**Beschreibung**

Die Erfindung geht aus von einer Vorrichtung zur Durchführung des Hämodialyseverfahrens nach dem Oberbegriff des Patentanspruchs 1. Eine derartige Vorrichtung ist aus der DE-OS 33 13 421 bekannt.

Die Verminderung des intravasalen Blutvolumens durch die Ultrafiltration in Verbindung mit den Änderungen der Ionenkonzentration im Plasma wird bei einer solchen Hämodialysevorrichtung für die Störungen des Kreislaufs und für einen Teil der unerwünschten Nebenwirkungen der intermittierenden Dialysebehandlung verantwortlich gemacht.

Die Entfernung des Flüssigkeitsüberschusses aus dem Körper des Patienten erfordert eine sehr präzise Steuerung der Flüssigkeitsbilanzierung, weshalb dieses Dialyseverfahren auch nur mit flüssigkeitsbilanzierenden Vorrichtungen durchgeführt werden kann. Trotz dieser präzisen Bilanzierung kommt es immer noch zu dialysetypischen Unannehmlichkeiten bei den Patienten, so zu Kopfschmerzen, Erbrechen und Muskelkrämpfen, was als "Disäquilibriumssyndrom" bezeichnet wird. Der Grund hierfür liegt vermutlich im zu raschen Entzug von Natriumionen aus dem Blut, der aufgrund der Konzentrationsdifferenz vom Natrium im Blut (extrakorporaler Kreislauf) und in der Dialysierflüssigkeit erfolgt.

Verschiedene Methoden wurden vorgeschlagen und erprobt, um das intravasale Blutvolumen kontinuierlich zu erfassen: Die kontinuierliche Messung des Hämatokrits durch Leitfähigkeitsmessung (Stiller, Mann; Biomed. Technik 25, 1980, 286-289 (Ergänzungsband)), die kontinuierliche Messung der Blutdichte (H. Holzer, u.a.; Wiener klinische Wochenschrift, 91. Jg., Heft 22, 1979, 762-765) und die Messung der Blutviskosität (R.N. Greenwood u.a.; Clinical Science, 1984, 66, 575-583).

Bei der von Stiller und Mann verwendeten Methode wird erstens die Bluttemperatur und Blutleitfähigkeit und zweitens die Leitfähigkeit eines durch Filtration des Blutes gewonnenen Filtrats kontinuierlich gemessen, aus den Meßwerten der Hämatokrit bzw. dessen Änderung über die Zeit berechnet und schließlich daraus auf die Veränderung des intravasalen Volumens geschlossen.

Dabei wird die Blutleitfähigkeit im arteriellen Blutschlauch mit einer Meßzelle gemessen. Zur Messung der Leitfähigkeit im Plasma wird kontinuierlich etwas Plasmawasser mit einem kleinen Hämofilter abgetrennt und durch eine zweite gleichartige Meßzellenanordnung geführt. Die Messung der Plasmaleitfähigkeit ist notwendig, da die Leitfähigkeit von Gesamtblut einerseits vom Hämatokrit und andererseits von der Elektrolytkonzentration im Plasma abhängt, die sich im Verlauf einer Dialysebehandlung in der Regel ändert.

Das von Stiller und Mann verwendete Verfahren hat zunächst den Nachteil, daß eine Leitfähigkeitszelle mit Temperatursensor in den extrakorporalen Kreislauf eingebracht werden muß. Das Verfahren hat aber ferner den Nachteil, daß für den genannten Meßzweck ein Filter im extrakorporalen Kreislauf angeordnet werden muß, um das für die Ermittlung des Filtratleitwertes notwendige Filtrat zu gewinnen.

Der Erfindung liegt die Aufgabe zugrunde, die eingangs erwähnte Vorrichtung zur Durchführung des Hämodialyseverfahren so fortzubilden, daß eine kontinuierliche Erfassung der Veränderung des intravasalen Blutvolumens bei der Hämodialyse ohne Verwendung eines Probeentnahmefilters ermöglicht wird.

Diese Aufgabe wird mit dem im Patentanspruch 1 ausgegebenen Mitteln gelöst.

Ausgestaltungen der Erfindung sind in den Unteransprüchen angegeben.

In einer Ausbildungsform ist es möglich, daß die Leitfähigkeit des Blutes zusätzlich stromab des Dialysators gemessen wird. Bei der Messung der Blutleitfähigkeit stromauf und stromab des Dialysators wird die Plasmaleitfähigkeit stromab des Dialysators errechnet, so daß aus den Hämatokritwerten stromauf und stromab des Dialysators und dem Blutfluß direkt die Ultrafiltrationsrate ermittelt werden kann.

Die kontinuierliche Erfassung der Veränderung des intravasalen Blutvolumens bei der Hämodialyse trägt entsprechend einer weiteren Ausgestaltung dazu bei, unerwünschte Nebenwirkungen zu verhindern, indem die Ultrafiltrationsrate bzw. -menge vom Ergebnis dieser Messung abhängig gemacht wird. Das bedeutet, daß beispielsweise die Ultrafiltration beendet wird, sobald das intravasale Volumen um einen bestimmten Prozentsatz abgenommen hat, bzw. die Ultrafiltrationsrate vermindert wird, sobald die Abnahme des intravasalen Blutvolumens in der Zeiteinheit einen bestimmten Betrag überschreitet.

Diese Einrichtung wird herangezogen, um die Ultrafiltrationseinrichtung des Hämodialysegerätes zu steuern. Sie kann aber auch als Schutzsystem dienen, das die Ultrafiltrationsrate und -menge und deren physiologische Auswirkung kontinuierlich erfaßt und bei der Überschreitung der festgelegten Grenze das Gerät in den sicheren Zustand überführt.

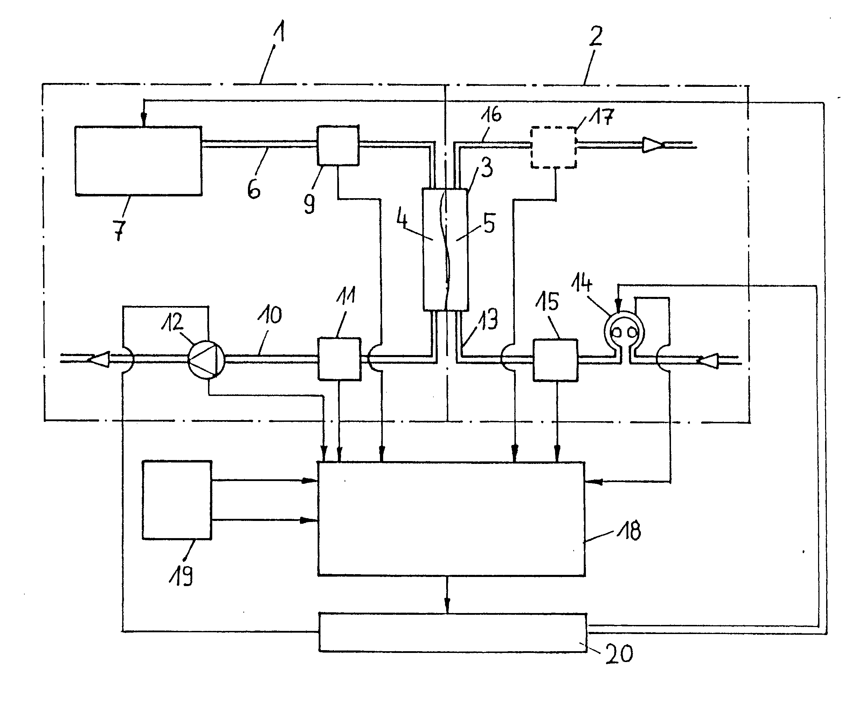

Anhand eines Ausführungsbeispiels soll die Erfindung näher erläutert werden. Dabei zeigt die beiliegende Figur die schematische Darstellung der Hämodialysevorrichtung. Die üblichen bekannten Bauteile der Hämodialysevorrichtung, die keine erfindungswesentliche Rolle spielen, wurden dabei weggelassen.

Die Hämodialysevorrichtung besteht im wesentlichen aus einem Dialysierflüssigkeitsteil 1 und einem extrakorporalen Kreislauf 2, zwischen denen sich ein Dialysator 3 mit einem Dialysierflüssigkeitskompartiment 4 und einem Blutkompartiment 5 befindet. Das Dialysierflüssigkeitskompartiment 4 ist stromauf des Dialysators 3 über eine Dialysierflüssigkeitsleitung 6 mit einer Dialysierflüssigkeitsmischeinrichtung 7 verbunden. In die Dialysierflüssigkeitsleitung 6 ist zwischen der Dialysierflüssigkeitsmischeinrichtung 7 und dem Dialysierflüssigkeitskompartiment 4 des Dialysators 3 eine erste Leitfähigkeitsmeßeinrichtung 9 angeordnet. Stromab des Dialysators 3 ist dem Dialysierflüssigkeitskompartiment 4 eine weitere Leitung 10 nachgeordnet, die eine zweite Leitfähigkeitsmeßeinrichtung 11, sowie eine Dialysierflüssigkeitspumpe 12 aufweist.

Im extrakorporalen Kreislauf 2 ist stromauf des Dialysators 3 in einer Blutleitung 13 eine Blutpumpe 14 und eine erste Leitfähigkeitsmeßeinrichtung 15 angeordnet.

Dem Blutkompartiment 5 ist stromab des Dialysators 3 eine weitere Blutleitung 16 angeordnet, die zusätzlich eine vierte Leitfähigkeitsmeßeinrichtung 17 aufweisen kann.

Die Dialysierflüssigkeitspumpe 12 und die Blutpumpe 14 sowie die Leitfähigkeitsmeßeinrichtungen 9, 11, 15 und 17 geben ihre Fördersignale, bzw. Meßwerte in eine Auswerteeinheit 18. Die Auswerteeinheit 18 steht weiterhin mit einer Eingabeeinheit 19 in Verbindung.

Weiterhin steht die Auswerteeinheit 18 mit einer Steuereinheit 20 in Verbindung, die ihre Signale an die Dialysierflüssigkeitsmischeinrichtung 7, die Dialysierflüssigkeitspumpe 12 und die Blutpumpe 14 abgibt.

In der Dialysierflüssigkeitsmischeinrichtung 7 ist eine nicht näher dargestellte Konzentratpumpe und ein entsprechender Wasserzulauf angeordnet, wobei die Zusammensetzung der Dialysierflüssigkeit entsprechend dem Ausgangssignal der Steuereinheit 20 entweder volumetrisch oder leitfähigkeitsproportional erfolgt.

Durch den durch die Dialysierflüssigkeitspumpe 12 in der Dialysierflüssigkeitsleitung 6 und 10 erzeugten Unterdruck wird die Dialysierflüssigkeit über den Dialysator 3 gefördert. Mit der in der Dialysierflüssigkeitsleitung 6 geschalteten erste Leitfähigkeitsmeßeinrichtung 9 wird die Leitfähigkeit der frischen Dialysierflüssigkeit gemessen. Der erhaltene Meßwert wird mit Hilfe einer in der ersten Leitfähigkeitsmeßeinrichtung 9 befindlichen Temperaturmeßeinrichtung kompensiert. Die zweite Leitfähigkeitsmeßeinrichtung 11 mißt die Leitfähigkeit der Dialysierflüssigkeit und des vom Blutkompartiment 5 in das Dialysierflüssigkeitskompartiment 4 des Dialysators 3 übergetretene Ultrafiltrat. Die zweite Leitfähigkeitsmeßeinrichtung 11 und auch die dritte und vierte Leitfähigkeitsmeßeinrichtung 15 und 17 sind ebenfalls wie die erste Leitfähigkeitsmeßeinrichtung 9 temperaturkompensiert ausgeführt und sind somit im Stande, die spezifische, temperaturkompensierte Leitfähigkeit der sie durchströmenden Flüssigkeiten zu ermitteln. Dabei wird jeweils die Leitfähigkeit von Vollblut vor bzw. nach Passieren des Blutkompartiments 5 des Dialysators 3 gemessen.

Der Dialysator 3 wird einerseits mit der Blutflußrate QB und andererseits mit der Dialysierflüssigkeitsflußrate QD durchströmt. Die Signale der Durchflußraten werden durch die Dialysierflüssigkeitspumpe 12 und die Blutpumpe 14 an die Auswerteeinheit 18 gegeben.

Der von der ersten Leitfähigkeitsmeßeinrichtung 9 gewonnene Meßwert wird mit CDi und der von der zweiten Leitfähigkeitsmeßeinrichtung 11 gewonnene Meßwert mit CDo bezeichnet. CBi und CBo sind die durch die Auswerteeinheit 18 zu ermitelnden Plasmaleitfähigkeitswerte im Blutkreislauf am Ort der dritten Leitfähigkeitsmeßzelle 15 (Bluteingang) bzw. der vierten Leitfähigkeitsmeßzelle 17 (Blutausgang).

Der Massentransfer von der Blut- zur Dialysierflüssigkeitsseite läßt sich bei einem idealen Dialysator aus der Konzentrationsdifferenz auf der Dialysierflüssigkeitsseite und dem Dialysierflüssigkeitsfluß errechnen:

$$dM = (CDo - CDi) \cdot QD \qquad (1)$$

Eine ähnliche Formel gilt für die Blutseite:

$$dM = (CBi - CBo) \cdot QB \qquad (2)$$

Aus (1) und (2) folgt:

$$CBi = CBo + \frac{(CDo - CDi) \cdot QD}{QB} \qquad (3)$$

Aufgrund des Gegenstromprinzips gilt:

3

$$CBo = CDi \qquad (4)$$

Mit (3) und (4) ergibt sich:

$$CBi = CDi + \frac{(CDo - CDi) \cdot QD}{QB} \qquad (5)$$

Es wurde damit gezeigt, daß unter idealisierten Bedingungen die Plasmaleitfähigkeit CBi aus der Leitfähigkeit der Dialysierflüssigkeit (CDi, CDo) und den Flüssen QD und QB errechnet werden kann.

Jedoch ist für einen realen Dialysator, wie er der Erfindung zugrunde liegt, die Dialysance und der Gibbs-Donnan-Koeffizient zu berücksichtigen.

Es gilt:

$$D = \frac{QB \cdot (CBi - CBo)}{CBi - CDi} \qquad (6)$$

bzw.

$$D = \frac{QD \cdot (CDo - CDi)}{CBi - CDi} \qquad (7)$$

Daraus ergibt sich:

$$CBi = CDi + \frac{(CDo - CDi) \cdot QD}{D} \qquad (8)$$

Die Dialysance D ist eine von Blut- und Dialysierflüssigkeitsfluß sowie vom Dialysatortyp abhängige Größe. Da Harnstoffdialysance und Na-Dialysance praktisch gleich groß sind und die Leitfähigkeit überwiegend durch die Natriumkonzentration bestimmt wird, kann für die Dialysance die Angaben für Harnstoff herangezogen werden.

Die Dialysanceangabe wird dabei über die Eingabeeinheit 19 in die Auswerteeinheit 18 eingegeben. Damit ist die Plasmaleitfähigkeit CBi durch die Auswerteeinheit 18 zu ermitteln. Die Natrium-Dialysance kann, wie weiter unten beschrieben, auch automatisch zu Beginn der Dialyse und gegebenenfalls auch im weiteren Verlauf ermittelt werden.

Zur Bestimmung der Veränderung des intravasalen Blutvolumens gilt:

$$\frac{dV}{V} = \frac{1 - HKt0}{HKt} \qquad (9)$$

wobei

$$HKt = HKt0 - a\,(dSB - b \cdot dSP) \qquad (10)$$

ist.

HKt0 ist der Hämatokrit am Beginn der Behandlung und wird ebenfalls über die Eingabeeinheit 19 in die Auswerteeinheit 18 eingegeben. HKt ist der gemessene bzw. berechnete Hämatokrit während der Behandlung.

dSB ist die Änderung der Blutleitfähigkeit und dSP die Änderung der Plasmaleitfähigkeit.

Entsprechend dem Gibbs-Donnan-Effekt sind a und b Konstante, nämlich

$a = 97,1 \pm 2$
$b = 1 \pm 0,03$

Diese Werte werden ebenfalls durch die Eingabeeinheit 19 in die Auswerteeinheit 18 eingegeben.

Die Änderung der Blutleitfähigkeit wird, wie erwähnt, mit Hilfe der dritten Leitfähigkeitsmeßeinrichtung 15 direkt gemessen. Die Änderung der Plasmaleitfähigkeit läßt sich nach Formel (8) errechnen, indem die Veränderung von CBi über die Zeit berücksichtigt wird.

HKt0 läßt sich am Beginn der Dialyse mit üblichen Verfahren ermitteln. Sind die Leitfähigkeitsmeßeinrichtungen 9, 11, 15 und 17 kalibriert, so kann aus Blut und Plasmaleitfähigkeit am Beginn der Dialyse auch HKt0 errechnet werden.

Bei der Ermittlung der Dialysance zur Berechnung der Plasmaleitfähigkeit CBi aus der bekannten Harnstoffclearance, welche der Dialysance bei CDi = 0 entspricht, muß berücksichtigt werden, daß der Verteilungsraum für Kationen im Blut der wässrige Anteil des Blutes außerhalb der Erythrozyten ist, während das Verteilungsvolumen für Harnstoff die gesamte wässrige Phase ist.

Da die Erythrozyten-Membran einen passiven Transport von Kationen zum Ausgleich von Konzentrationsdifferenzen nicht zuläßt, muß ferner die Veränderung des Erythrozytenvolumens aufgrund von osmotischen Effekten berücksichtigt werden.

In Abhängigkeit von der Veränderung des intravasalen Blutvolumens, welches nach Formel (9) errechnet wurde, kann die Ultrafiltrationsrate bestimmt werden.

Wird zusätzlich die Blutleitfähigkeit mit Hilfe der Leitfähigkeitsmeßeinrichtung 17 stromab des Dialysators gemessen und mit Hilfe von Formel (6) die Plasmaleitfähigkeit CBo stromab des Dialysators 3 errechnet, so kann aus den HKt-Werten stromauf und stromab des Dialysators 3 und dem Blutfluß direkt die Ultrafiltrationsrate ermittelt werden.

Entsprechend den ermittelten Werten für die Veränderung des intravasalen Blutvolumens erhält die Steuereinheit 20 die entsprechenden Befehle und diese gibt ihre Signale an die Dialysierflüssigkeitsmischeinrichtung 7, die Dialysierflüssigkeitspumpe 12 bzw. die Blutpumpe 14. Damit wird die Zusammensetzung der Dialysierflüssigkeit sowie die Pumprate der Dialysierflüssigkeit und die Blutfördermenge entsprechend geregelt.

Die Natrium-Dialysance kann wie folgt ermittelt werden: Stromauf des Dialysators wird blutseitig (arteriell) ein Bolus einer hypertonen NaCl-Lösung in das Blutschlauchsystem eingespritzt. Dadurch verändert sich die Leitfähigkeitsdifferenz auf der Dialysierflüssigkeitsseite für den Zeitraum der Bolusapplikation bei Vernachlässigung der Laufzeitdifferenzen.

Das Integral über diese Leitfähigkeitsveränderung geteilt durch das Integral der Leitfähigkeitsveränderung gemessen mit Hilfe des im Blutkreislauf angeordneten Leitfähigkeitssensors (15 bzw. 17) ergibt dann direkt die Dialysance.

**Patentansprüche**

1. Vorrichtung zur Durchführung eines Hämodialyseverfahrens, aufweisend einen Dialysator (3), der durch eine Membran in zwei Kammern geteilt ist, dessen erste Kammer (4) in einen Dialysierflüssigkeitsweg und dessen zweite Kammer (5) in einen Blutweg geschaltet ist, der Dialysierflüssigkeitsweg aufweisend stromauf des Dialysators (3) wenigstens eine Dialysierflüssigkeitsmischeinrichtung (7) und eine erste Leitfähigkeitsmeßeinrichtung (9) und stromab des Dialysators wenigstens eine Dialysierflüssigkeitspumpe (12), und der Blutweg wenigstens eine Blutpumpe (14) aufweisend, dadurch gekennzeichnet, daß stromab des Dialysators (3) im Dialysierflüssigkeitsweg eine zweite Leitfähigkeitsmeßeinrichtung (11) und daß stromauf des Dialysators (3) im Blutweg (13) eine dritte Leitfähigkeitsmeßeinrichtung (15) angeordnet ist, daß die Leitfähigkeitsmeßeinrichtungen (9, 11, 15) mit einer Auswerteeinheit (18) verbunden sind, welche in der Weise ausgebildet ist, daß aus den Meßwerten der Leitfähigkeitsmeßeinrichtungen (9, 11, 15) sowie aus dem Blutfluß und aus Leistungsparametern des Dialysators die Plasmaleitfähigkeit, sowie die Änderung der Plasma- und Blutleitfähigkeit ermittelt wird, aus der Änderung der Plasma- und Blutleitfähigkeit der Hämatokrit während der Dialyse ermittelt wird, und aus dem Hämatokrit auf die Veränderung des intravasalen Blutvolumens geschlossen wird.

2. Vorrichtung nach Anspruch 1, dadurch gekennzeichnet, daß eine vierte Leitfähigkeitsmeßeinrichtung (17) stromab des Dialysators (3) im Blutweg (16) angeordnet und mit der Auswerteeinheit (18) verbunden ist.

3. Vorrichtung nach den Ansprüchen 1 oder 2, dadurch gekennzeichnet, daß die Leistungsparameter des Dialysators (3) und sonstige Konstanten über eine Eingabeeinheit (19) in die Auswerteeinheit (18) eingegeben werden.

4. Vorrichtung nach einem der Ansprüche 1-3, dadurch gekennzeichnet, daß die Auswerteeinheit (18) mit einer Steuereinheit (20) zur Steuerung der Dialysierflüssigkeitspumpe (12), der Blutpumpe (14) und/oder der Dialysierflüssigkeitsmischeinrichtung (7) verbunden ist.

## Claims

1. Apparatus for carrying out a hemodialysis comprising a dialyser (3) which is divided by a membrane into two chambers, the first chamber (4) of which is connected into a dialysis solution path and the second chamber (5) of which is connected into a blood path, the dialysis solution path comprising upstream of the dialyzer (3) at least one dialysis solution mixing means (7) and a first conductivity measuring means (9) and downstream of the dialyzer at least one dialysis solution pump (12) and the blood path comprising at least one blood pump (14), characterized in that downstream of the dialyzer (3) in the dialysis solution path a second conductivity measuring means (11) is arranged and upstream of the dialyzer (3) in the blood path (13) a third conductivity measuring means (15) is arranged, that the conductivity measuring means (9, 11, 15) are connected to an evaluating unit (18) which from the measured values of the conductivity measuring means (9, 11, 15) and from the blood flow and the performance parameters of the dialyzer determine the plasma conductivity and the change in the plasma and blood conductivity, from the change in the plasma and blood conductivity determine the hematocrit during the dialysis and from the hematocrit deduce the change in the intravascular blood volume.

2. Apparatus according to claim 1, characterized in that a fourth conductivity measuring means (17) is disposed downstream of the dialyzer (3) in the blood path (16) and is connected to the evaluating unit (18).

3. Apparatus according to claim 1 or 2, characterized in that the performance parameters of the dialyzer (3) and other constants can be entered via an input unit (19) into the evaluating unit (18).

4. Apparatus according to any of claims 1 - 3, charaterized in that the evaluating unit (18) is connected to a control unit (20) for controlling the dialysis solution pump (12), the blood pump (14) and/or the dialysis solution mixing means (7).

## Revendications

1. Dispositif pour la mise en oeuvre d'un procédé d'hémodialyse, comportant un dialyseur (3) qui est partagé, par une membrane, en deux chambres dont la première chambre (4) est montée dans un parcours de liquide de dialyse et dont la deuxième chambre (5) est montée dans un parcours sanguin, le parcours de liquide de dialyse comportant en amont du dialyseur (3) au moins un dispositif de mélange de liquide de dialyse (7) et un premier dispositif de mesure de conductibilité (9) et en aval du dialyseur au moins une pompe de liquide de dialyse (12), et le parcours sanguin comportant au moins une pompe à sang (14), caractérisé en ce qu'un deuxième dispositif de mesure de conductibilité (11) est monté dans le parcours de liquide de dialyse, en aval du dialyseur (3) et un troisième dispositif de mesure de conductibilité (15) est monté dans le parcours sanguin (13), en amont du dialyseur (3), en ce que les dispositifs de mesure de conductibilité (9, 11, 15) sont reliés à une unité d'exploitation (18) qui est conçue de manière à déterminer la conductibilité du plasma ainsi que la variation de la conductibilité du plasma et du sang, à partir des valeurs mesurées par les dispositifs de mesure de conductibilité (9, 11, 15) ainsi qu'à partir du flux sanguin et des paramètres de rendement du dialyseur, à déterminer l'hématocrite pendant la dialyse et de manière à déterminer la variation du volume sanguin intravasculaire, à partir de l'hématocrite.

2. Dispositif selon la revendication 1, caractérisé en ce qu'un quatrième dispositif de mesure de conductibilité (17) est placé dans le parcours sanguin (16), en aval du dialyseur (3) et est relié à l'unité d'exploitation (18).

3. Dispositif selon les revendications 1 ou 2, caractérisé en ce que les paramètres de rendement du dialyseur (3) et diverses constantes sont introduites dans l'unité d'exploitation (18), par une unité de saisie (19).

4. Dispositif selon l'une des revendications 1 à 3, caractérisé en ce que l'unité d'exploitation (18) est reliée à une unité de commande (20) en vue de commander la pompe du liquide de dialyse (12), la pompe du sang (14) et/ou le dispositif de mélange de liquide de dialyse (7).